# EUROPEAN PATENT APPLICATION

(11) **EP 4 480 416 A1**
(43) Date of publication of application: **25.12.2024**
(21) Application number: 23756515.5
(22) Date of filing: 09.01.2023
(51) Int. Cl.: A61B 5/349, A61B 5/339, A61B 5/327, G16H 50/20

(54) **ELECTROCARDIOGRAM DELINEATION AND RESULT INTEGRATION METHOD**

(30) Priority: 18.02.2022 KR 20220021177
(71) Applicant: VUNO INC., Seoul 06541 (KR)
(72) Inventor: KIM, Kyung Geun, Seoul 06288 (KR); JOO, Sunghoon, Seoul 04778 (KR)
(74) Representative: AWA Sweden AB
(86) International application number: PCT/KR2023/000374
(87) International publication number: WO 2023/158102

(57) **Abstract**

According to an embodiment of the present disclosure, a method for processing medical data and a computing device using the same. Specifically, according to the present disclosure, a computing device receives one or more ECG data, generates ECG delineation information corresponding to a plurality of samples generated from the one or more received ECG data through the ECG delineation model, and integrates the plurality of ECG delineation information.

## Description

### [Technical Field]

The present disclosure relates to a method of processing medical data, and more particularly, to a method of generating ECG delineation information for ECG data measured from one or more leads, and integrating the ECG delineation information.

### [Background Art]

A waveform of electrocardiogram (ECG), which reflects an electrical activation stage of the heart, is basically composed of detailed areas of a P-wave, QRS-complex, and T-wave, the P wave of each heartbeat occurs at the time of atrial depolarization, the QRS complex reflects the time of ventricular depolarization, and the T wave reflects the time of ventricular repolarization.

ECG delineation is a signal processing process that separates the detailed areas described above from ECG data. In electrocardiogram analysis, the electrocardiogram delineation occupies a very important position, and the reason is that after obtaining detailed characteristics of the heartbeat through an electrocardiogram delineation result, information on relative time intervals such as the amplitude, PR-interval, and ST-interval, and QT-interval of detailed areas obtained to determine abnormalities in heartbeat rhythm or presence of heart disease.

However, as the types of ECG measurement equipment are diversified (Standard 12-Lead, Holter, Mobile single-lead, Limb lead), the type and number of leads for measuring ECG and the length of measurement time are different, so there are differences between the data received from each equipment, making it difficult to stably derive the electrocardiogram delineation information.

US Patent Publication No. 2021-0219920 discloses a system and method for obtaining an internal electrocardiogram.

### [Disclosure]

### [Technical Problem]

The present disclosure is contrived in response to the above-mentioned background technology, and has been made in an effort to provide a method of deriving stable ECG delineation information regardless of the number of leads of the measurement equipment and the length of measurement time.

### [Technical Solution]

In order to implement the object, according to an exemplary embodiment of the present disclosure, disclosed is an ECG delineation method performed by one or more processors of a computing device. The method may include: receiving one or more ECG data; generating ECG delineation information corresponding to a plurality of samples having predetermined time lengths, from the one or more received ECG data through an ECG delineation model; and integrating the plurality of ECG delineation information.

In an alternative embodiment, the receiving of the ECG data may include receiving the one or more ECG data from one of a plurality of different types of ECG measurement equipment.

In an alternative embodiment, the generating of the ECG delineation information corresponding to the plurality of samples having the predetermined time lengths, from the one or more ECG data through the ECG delineation model may include confirming the number of leads and the measurement time length of the ECG measurement equipment, generating the plurality of samples by dividing the one or more ECG data based on the predetermined time length, and generating respective ECG delineation information corresponding to the plurality of samples through the ECG delineation model.

In an alternative embodiment, the predetermined time length may be determined independently of the number of leads or a measurement time length of the ECG measurement equipment.

In an alternative embodiment, the ECG delineation information may include at least one of location information of a P-wave, location information of a QRS-complex, or location information of a T-wave, and the processor may derive, from the ECG delineation information, at least one of amplitude information of the P-wave, amplitude information of the QRS-complex, or amplitude information of the T-wave.

In an alternative embodiment, the integrating of the plurality of ECG delineation information may include generating smoothed ECG delineation information for the plurality of samples, and integrating the smoothed ECG delineation information for the plurality of samples.

In an alternative embodiment, the generating of the smoothed ECG delineation information for the plurality of samples may further include deriving a representative value among result values of the ECG delineation information for the plurality of samples within a fixed time interval.

In an alternative embodiment, the integrating of the smoothed ECG delineation information for the plurality of samples may further include deriving a representative value among the result values of the smoothed ECG delineation information.

In an alternative embodiment, the present disclosure may further include extracting ECG characteristics from the integrated ECG delineation information. The extracting of ECG characteristics from the integrated ECG delineation information may include dividing the integrated ECG delineation information based on one heart beat time, extracting each ECG characteristic from the ECG delineation information divided based on the one heart beat time, and deriving a representative value of each extracted ECG characteristic.

In an alternative embodiment, the ECG characteristics include at least one of an RR-interval, a QRS duration, a QT interval, a QT-corrected interval, a PR-segment; a PR-interval, an ST-segment, or a TP-segment.

In an alternative embodiment, the ECG delineation method according to the present disclosure may further include: generating a user interface (UI) including information on the ECG delineation information, and the user interface may include a first area displaying ECG data measured by the ECG measurement equipment, a second area displaying the ECG delineation information, and a third area displaying a value of the ECG characteristic.

According to an alternative embodiment of the present disclosure, disclosed is a computer program stored in a computer readable storage medium, which includes instructions which allow a computing device to perform operations. The operations may include: an operation of receiving one or more ECG data; an operation of generating ECG delineation information corresponding to a plurality of samples having predetermined time lengths, from the one or more received ECG data through an ECG delineation model; and an operation of integrating the plurality of ECG delineation information.

According to an alternative embodiment of the present disclosure, disclosed is a computing device. The computing device may include: a processor including one or more cores; a network unit receiving one or more ECG data; and a memory, and among them, the processor may include a process of receiving one or more ECG data, generating ECG delineation information corresponding to a plurality of samples having predetermined time lengths, from the one or more ECG data through an ECG delineation model, and integrating the ECG delineation information.

### [Advantageous Effects]

The present disclosure can stably provide ECG delineation information regardless of the number of leads and the length of measurement time.

### [Description of Drawings]

The following accompanying drawings are only some of the embodiment of the present disclosure so as to be used for describing the embodiment of the present disclosure, and in the technical field of the present disclosure, those (hereinafter, referred to as "normal technician") skilled in the technical field of the present disclosure can obtain other drawings based on the drawings without an effort to reach a new invention.
FIG. 1 is a block diagram of a computing device for performing an operation for generating ECG delineation information according to an embodiment of the present disclosure.
FIG. 2 is a schematic view illustrating a network function according to an embodiment of the present disclosure.
FIG. 3 is a flowchart of a method for generating and integrating ECG delineation information according to an embodiment of the present disclosure.
FIG. 4 is a block diagram illustrating a process of generating ECG delineation information by a computing device according to an embodiment of the present disclosure.
FIG. 5 is a conceptual diagram illustrating a smoothing process of the ECG delineation information according to an embodiment of the present disclosure.
FIG. 6 is a conceptual diagram illustrating ECG characteristics according to an embodiment of the present disclosure.
FIG. 7 is a block diagram of a computing device according to an embodiment of the present disclosure.

### [Best Mode]

The present disclosure discloses a method for generating ECG delineation information stably regardless of the number of leads and the length of measurement time, by receiving ECG data of a patient measured through ECG measurement equipment, and deriving an integrated result to extract ECG characteristics.

Various exemplary embodiments will now be described with reference to drawings. In the present specification, various descriptions are presented to provide appreciation of the present disclosure. However, it is apparent that the exemplary embodiments can be executed without the specific description.

"Component", "module", "system", and the like which are terms used in the specification refer to a computer-related entity, hardware, firmware, software, and a combination of the software and the hardware, or execution of the software. For example, the component may be a processing procedure executed on a processor, the processor, an object, an execution thread, a program, and/or a computer, but is not limited thereto. For example, both an application executed in a computing device and the computing device may be the components. One or more components may reside within the processor and/or a thread of execution. One component may be localized in one computer. One component may be distributed between two or more computers. Further, the components may be executed by various computer-readable media having various data structures, which are stored therein. The components may perform communication through local and/or remote processing according to a signal (for example, data transmitted from another system through a network such as the Internet through data and/or a signal from one component that interacts with other components in a local system and a distribution system) having one or more data packets, for example.

The term "or" is intended to mean not exclusive "or" but inclusive "or". That is, when not separately specified or not clear in terms of a context, a sentence "X uses A or B" is intended to mean one of the natural inclusive substitutions. That is, the sentence "X uses A or B" may be applied to any of the case where X uses A, the case where X uses B, or the case where X uses both A and B. Further, it should be understood that the term "and/or" used in this specification designates and includes all available combinations of one or more items among enumerated related items.

It should be appreciated that the term "comprise" and/or "comprising" means presence of corresponding features and/or components. However, it should be appreciated that the term "comprises" and/or "comprising" means that presence or addition of one or more other features, components, and/or a group thereof is not excluded. Further, when not separately specified or it is not clear in terms of the context that a singular form is indicated, it should be construed that the singular form generally means "one or more" in this specification and the claims.

The term "at least one of A or B" should be interpreted to mean "a case including only A", "a case including only B", and "a case in which A and B are combined".

Those skilled in the art need to recognize that various illustrative logical blocks, configurations, modules, circuits, means, logic, and algorithm steps described in connection with the exemplary embodiments disclosed herein may be additionally implemented as electronic hardware, computer software, or combinations of both sides. To clearly illustrate the interchangeability of hardware and software, various illustrative components, blocks, configurations, means, logic, modules, circuits, and steps have been described above generally in terms of their functionalities. Whether the functionalities are implemented as the hardware or software depends on a specific application and design restrictions given to an entire system. Skilled artisans may implement the described functionalities in various ways for each particular application. However, such implementation decisions should not be interpreted as causing a departure from the scope of the present disclosure.

The description of the presented exemplary embodiments is provided so that those skilled in the art of the present disclosure use or implement the present disclosure. Various modifications to the exemplary embodiments will be apparent to those skilled in the art. Generic principles defined herein may be applied to other embodiments without departing from the scope of the present disclosure. Therefore, the present disclosure is not limited to the exemplary embodiments presented herein. The present disclosure should be analyzed within the widest range which is coherent with the principles and new features presented herein.

In the present disclosure, a network function and an artificial neural network and a neural network may be interchangeably used.

Meanwhile, the term "image" or "image data" used throughout the detailed description and claims of the present disclosure refers to multi-dimensional data constituted by discrete image elements (e.g., pixels in a 2D image), and in other words, refers to an object which may be seen with an eye (e.g., displayed on a video screen) or a digital representation of the object (such as a file corresponding to a pixel output of CT, MRI detector, etc.).

For example, the "image" may be computed tomography (CT), magnetic resonance imaging (MRI), ultrasonic waves, a medical image of a subject collected by any other medical imaging system known in the technical field of the present disclosure. The image may not particularly be provided in a medical context, and may be provided in a non-medical context, and may be for example, a security search X-ray imaging.

Throughout the detailed description and claims of the present disclosure, a `Digital Imaging and Communications in Medicine (DICOM)' standard is a term which collectively refers to several standards used for digital image representation and communication in a medical device, so that the DICOM standard is announced by the Federation Committee, constituted in the American College Radiology (ACR) and the National Electrical Manufacturers Association (NEMA).

Further, throughout the detailed description and claims of the present disclosure, a Picture Archiving and Communication System (PACS)' is a term that refers to a system for performing storing, processing, and transmitting according to the DICOM standard, and medical images obtained by using digital medical image equipment such as X-ray, CT, and MRI may be stored in a DICOM format and transmitted to terminals inside or outside a hospital through a network, and additionally include a reading result and a medical chart.

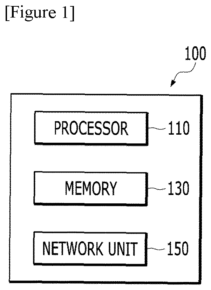

FIG. 1 is a block diagram of a computing device for generating ECG 'result'? delineation information according to an embodiment of the present disclosure.

A configuration of the computing device 100 illustrated in FIG. 1 is only an example shown through simplification. In an exemplary embodiment of the present disclosure, the computing device 100 may include other components for performing a computing environment of the computing device 100 and only some of the disclosed components may constitute the computing device 100.

The computing device 100 may include a processor 110, a memory 130, and a network unit 150.

The processor 110 may be constituted by one or more cores and may include processors for data analysis and deep learning, which include a central processing unit (CPU), a general purpose graphics processing unit (GPGPU), a tensor processing unit (TPU), and the like of the computing device. The processor 110 may read a computer program stored in the memory 130 to perform data processing for machine learning according to an exemplary embodiment of the present disclosure. According to an exemplary embodiment of the present disclosure, the processor 110 may perform a calculation for learning the neural network. The processor 110 may perform calculations for learning the neural network, which include processing of input data for learning in deep learning (DL), extracting a feature in the input data, calculating an error, updating a weight of the neural network using backpropagation, and the like. At least one of the CPU, GPGPU, and TPU of the processor 110 may process learning of a network function. For example, both the CPU and the GPGPU may process the learning of the network function and data classification using the network function. Further, in an exemplary embodiment of the present disclosure, processors of a plurality of computing devices may be used together to process the learning of the network function and the data classification using the network function. Further, the computer program executed in the computing device according to an exemplary embodiment of the present disclosure may be a CPU, GPGPU, or TPU executable program.

The processor 110 may receive one or more ECG data from a plurality of different types of ECG measurement equipment and generate ECG delineation information. For example, the processor 110 may receive one or more ECG data from a plurality of ECG measurement equipment having different numbers of leads or different measurement time lengths, and generate ECG delineation information for each ECG measurement equipment.

In an alternative embodiment, the processor divides the electrocardiogram data based on a 'predetermined length of time, determined independently of the number of leads of the electrocardiogram measurement equipment or the measurement time length to generate a plurality of samples having predetermined time lengths corresponding to the ECG data. In this case, the predetermined time length may be a length with a size to be received as an input by an ECG delineation model.

The processor 110 may input the plurality of divided samples having the predetermined time lengths, respectively into the ECG delineation model, and obtain the ECG delineation information generated for the respective samples, as an output. The input of the ECG delineation model may be ECG data having one fixed length, i.e., a predetermined time length. Further, the output of the ECG delineation model may be information include the ECG delineation information, i.e., location information of the P wave, QRS complex, and T wave of all beats included in the plurality of input samples. Here, 'correspondence' may mean that one piece of ECG delineation information is obtained from each of the plurality of samples, so that each sample and the ECG delineation information satisfy a one-to-one correspondence relationship. At this time, the number of output ECG delineation information may be equal to the number of plurality of input samples, each having the predetermined time length.

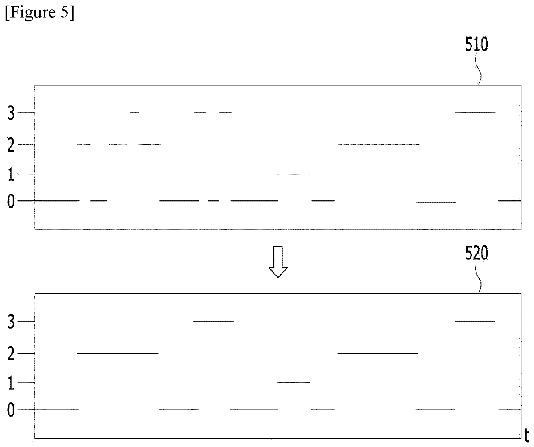

The processor 110 may integrate the ECG delineation information for the plurality of samples having the predetermined time lengths, respectively into one ECG delineation information. For example, the ECG delineation information for the plurality of samples may be smoothed, and then a mode at a specific time point may be used as a value of the integrated ECG delineation information, but the present disclosure is not limited thereto. A detailed description of smoothing will be described later with reference to FIG. 5.

The processor 110 may extract ECG characteristics based on the integrated ECG delineation information. The ECG characteristics may include RR-interval, QRS duration, QT interval, or QT-corrected interval, which is the basis for determining heartbeat abnormalities and heart disease, but the present disclosure is not limited thereto. Since the integrated ECG delineation information includes multiple heartbeats, in order to extract the ECG characteristics, it is necessary to generate ECG delineation information divided by one heartbeat time.

The processor 110 may divide the integrated ECG delineation information based on one heart beat time, extract each ECG characteristic from the ECG delineation information divided by each generated one heart beat time, and then derive a representative value of each characteristic.

According to an embodiment of the present disclosure, the memory 130 may store any type of information generated or determined by the processor 110 or any type of information received by the network unit 150.

According to an exemplary embodiment of the present disclosure, the memory 130 may include at least one type of storage medium of a flash memory type storage medium, a hard disk type storage medium, a multimedia card micro type storage medium, a card type memory (for example, an SD or XD memory, or the like), a random access memory (RAM), a static random access memory (SRAM), a read-only memory (ROM), an electrically erasable programmable read-only memory (EEPROM), a programmable read-only memory (PROM), a magnetic memory, a magnetic disk, and an optical disk. The computing device 100 may operate in connection with a web storage performing a storing function of the memory 130 on the Internet. The description of the memory is just an example and the present disclosure is not limited thereto.

The network unit 150 according to several embodiments of the present disclosure may use various wired communication systems, such as a Public Switched Telephone Network (PSTN), an x Digital Subscriber Line (xDSL), a Rate Adaptive DSL (RADSL), a Multi Rate DSL (MDSL), a Very High Speed DSL (VDSL), a Universal Asymmetric DSL (UADSL), a High Bit Rate DSL (HDSL), and a local area network (LAN).

The network unit 150 presented in the present specification may use various wireless communication systems, such as Code Division Multi Access (CDMA), Time Division Multi Access (TDMA), Frequency Division Multi Access (FDMA), Orthogonal Frequency Division Multi Access (OFDMA), Single Carrier-FDMA (SC-FDMA), and other systems.

The network unit 150 according to an exemplary embodiment of the present disclosure may use an arbitrary type known wired/wireless communication systems.

The techniques described herein may be used in other networks in addition to those mentioned above.

The network unit 150 may receive medical images representing body organs from a medical imaging system. For example, the medical image representing the body organ may be data for training or inference of a neural network model that learns two-dimensional features or three-dimensional features. The medical image with the body organ representation may be a three-dimensional T1 MR image comprising at least one brain region. The medical image representing the body organ may include any image associated with the body organ obtained through imaging, such as an X-ray image, a CT image, or the like, without limitation to the foregoing examples.

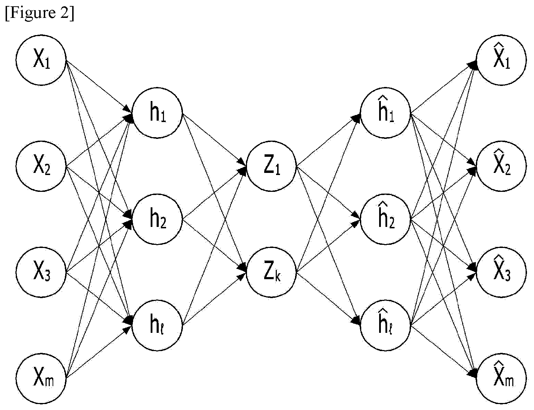

FIG. 2 is a schematic diagram illustrating a network function according to an exemplary embodiment of the present disclosure.

Throughout the present specification, a computation model, the neural network, a network function, and the neural network may be used as the same meaning. The neural network may be generally constituted by an aggregate of calculation units which are mutually connected to each other, which may be called nodes. The nodes may also be called neurons. The neural network is configured to include one or more nodes. The nodes (alternatively, neurons) constituting the neural networks may be connected to each other by one or more links.

In the neural network, one or more nodes connected through the link may relatively form the relationship between an input node and an output node. Concepts of the input node and the output node are relative and a predetermined node which has the output node relationship with respect to one node may have the input node relationship in the relationship with another node and vice versa. As described above, the relationship of the input node to the output node may be generated based on the link. One or more output nodes may be connected to one input node through the link and vice versa.

In the relationship of the input node and the output node connected through one link, a value of data of the output node may be determined based on data input in the input node. Here, a link connecting the input node and the output node to each other may have a weight. The weight may be variable and the weight is variable by a user or an algorithm in order for the neural network to perform a desired function. For example, when one or more input nodes are mutually connected to one output node by the respective links, the output node may determine an output node value based on values input in the input nodes connected with the output node and the weights set in the links corresponding to the respective input nodes.

As described above, in the neural network, one or more nodes are connected to each other through one or more links to form a relationship of the input node and output node in the neural network. A characteristic of the neural network may be determined according to the number of nodes, the number of links, correlations between the nodes and the links, and values of the weights granted to the respective links in the neural network. For example, when the same number of nodes and links exist and there are two neural networks in which the weight values of the links are different from each other, it may be recognized that two neural networks are different from each other.

The neural network may be constituted by a set of one or more nodes. A subset of the nodes constituting the neural network may constitute a layer. Some of the nodes constituting the neural network may constitute one layer based on the distances from the initial input node. For example, a set of nodes of which distance from the initial input node is n may constitute n layers. The distance from the initial input node may be defined by the minimum number of links which should be passed through for reaching the corresponding node from the initial input node. However, a definition of the layer is predetermined for description and the order of the layer in the neural network may be defined by a method different from the aforementioned method. For example, the layers of the nodes may be defined by the distance from a final output node.

The initial input node may mean one or more nodes in which data is directly input without passing through the links in the relationships with other nodes among the nodes in the neural network. Alternatively, in the neural network, in the relationship between the nodes based on the link, the initial input node may mean nodes which do not have other input nodes connected through the links. Similarly thereto, the final output node may mean one or more nodes which do not have the output node in the relationship with other nodes among the nodes in the neural network. Further, a hidden node may mean nodes constituting the neural network other than the initial input node and the final output node.

In the neural network according to an exemplary embodiment of the present disclosure, the number of nodes of the input layer may be the same as the number of nodes of the output layer, and the neural network may be a neural network of a type in which the number of nodes decreases and then, increases again from the input layer to the hidden layer. Further, in the neural network according to another exemplary embodiment of the present disclosure, the number of nodes of the input layer may be smaller than the number of nodes of the output layer, and the neural network may be a neural network of a type in which the number of nodes decreases from the input layer to the hidden layer. Further, in the neural network according to yet another exemplary embodiment of the present disclosure, the number of nodes of the input layer may be larger than the number of nodes of the output layer, and the neural network may be a neural network of a type in which the number of nodes increases from the input layer to the hidden layer. The neural network according to still yet another exemplary embodiment of the present disclosure may be a neural network of a type in which the neural networks are combined.

A deep neural network (DNN) may refer to a neural network that includes a plurality of hidden layers in addition to the input and output layers. When the deep neural network is used, the latent structures of data may be determined. That is, latent structures of photos, text, video, voice, and music (e.g., what objects are in the photo, what the content and feelings of the text are, what the content and feelings of the voice are) may be determined. The deep neural network may include a convolutional neural network (CNN), a recurrent neural network (RNN), an auto encoder, generative adversarial networks (GAN), a restricted Boltzmann machine (RBM), a deep belief network (DBN), a Q network, a U network, a Siam network, a Generative Adversarial Network (GAN), and the like. The description of the deep neural network described above is just an example and the present disclosure is not limited thereto.

In an exemplary embodiment of the present disclosure, the network function may include the auto encoder. The auto encoder may be a kind of artificial neural network for outputting output data similar to input data. The auto encoder may include at least one hidden layer and odd hidden layers may be disposed between the input and output layers. The number of nodes in each layer may be reduced from the number of nodes in the input layer to an intermediate layer called a bottleneck layer (encoding), and then expanded symmetrical to reduction to the output layer (symmetrical to the input layer) in the bottleneck layer. The auto encoder may perform non-linear dimensional reduction. The number of input and output layers may correspond to a dimension after preprocessing the input data. The auto encoder structure may have a structure in which the number of nodes in the hidden layer included in the encoder decreases as a distance from the input layer increases. When the number of nodes in the bottleneck layer (a layer having a smallest number of nodes positioned between an encoder and a decoder) is too small, a sufficient amount of information may not be delivered, and as a result, the number of nodes in the bottleneck layer may be maintained to be a specific number or more (e.g., half of the input layers or more).

The neural network may be learned in at least one scheme of supervised learning, unsupervised learning, semi supervised learning, or reinforcement learning. The learning of the neural network may be a process in which the neural network applies knowledge for performing a specific operation to the neural network.

The neural network may be learned in a direction to minimize errors of an output. The learning of the neural network is a process of repeatedly inputting learning data into the neural network and calculating the output of the neural network for the learning data and the error of a target and back-propagating the errors of the neural network from the output layer of the neural network toward the input layer in a direction to reduce the errors to update the weight of each node of the neural network. In the case of the supervised learning, the learning data labeled with a correct answer is used for each learning data (i.e., the labeled learning data) and in the case of the unsupervised learning, the correct answer may not be labeled in each learning data. That is, for example, the learning data in the case of the supervised learning related to the data classification may be data in which category is labeled in each learning data. The labeled learning data is input to the neural network, and the error may be calculated by comparing the output (category) of the neural network with the label of the learning data. As another example, in the case of the unsupervised learning related to the data classification, the learning data as the input is compared with the output of the neural network to calculate the error. The calculated error is back-propagated in a reverse direction (i.e., a direction from the output layer toward the input layer) in the neural network and connection weights of respective nodes of each layer of the neural network may be updated according to the back propagation. A variation amount of the updated connection weight of each node may be determined according to a learning rate. Calculation of the neural network for the input data and the back-propagation of the error may constitute a learning cycle (epoch). The learning rate may be applied differently according to the number of repetition times of the learning cycle of the neural network. For example, in an initial stage of the learning of the neural network, the neural network ensures a certain level of performance quickly by using a high learning rate, thereby increasing efficiency and uses a low learning rate in a latter stage of the learning, thereby increasing accuracy.

In learning of the neural network, the learning data may be generally a subset of actual data (i.e., data to be processed using the learned neural network), and as a result, there may be a learning cycle in which errors for the learning data decrease, but the errors for the actual data increase. Overfitting is a phenomenon in which the errors for the actual data increase due to excessive learning of the learning data. For example, a phenomenon in which the neural network that learns a cat by showing a yellow cat sees a cat other than the yellow cat and does not recognize the corresponding cat as the cat may be a kind of overfitting. The overfitting may act as a cause which increases the error of the machine learning algorithm. Various optimization methods may be used in order to prevent the overfitting. In order to prevent the overfitting, a method such as increasing the learning data, regularization, dropout of omitting a part of the node of the network in the process of learning, utilization of a batch normalization layer, etc., may be applied.

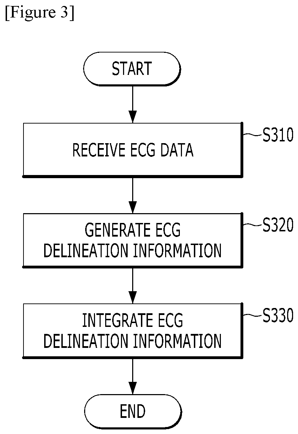

FIG. 3 is a flowchart of a method for generating and integrating ECG delineation information according to an embodiment of the present disclosure.

In step S310, the processor 110 of the computing device 100 may receive, from the ECG measurement equipment, at least one ECG data constituted by electrodes connected and one or more leads calculated therefrom. At this time, ECG data may refer to data received from electrodes of the ECG measurement device to the ECG measurement device, or may refer to data received from electrodes directly connected to the computing device 100.

In step S320, the processor 110 may divide the ECG data received in step S310 into a plurality of samples having predetermined lengths, and generate ECG delineation information for the plurality of respective divided samples. Here, the lengths of the plurality of samples may be lengths which may be analyzed by the ECG delineation model for generating the ECG delineation information.

In step S330, the processor 110 may generate single ECG delineation information by integrating the ECG delineation information of the plurality of divided samples into one. At this time, the processor 110 may generate the single ECG delineation information after performing a smoothing process on each of the divided samples. A specific process of smoothing will be described later with reference to FIG. 5.

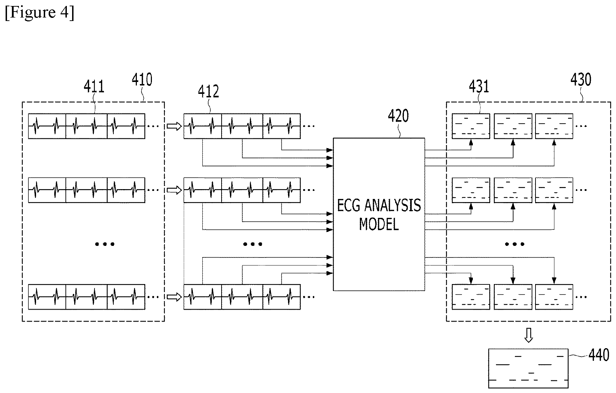

FIG. 4 is a block diagram illustrating a process of generating ECG delineation information by a computing device according to an embodiment of the present disclosure.

ECG data 411 measured by each of the one or more leads is received from one of a plurality of different types of electrocardiogram measurement equipment. A task may be performed which divides all of the received ECG data 410 into lengths that may be analyzed by the ECG delineation model 420 to generate a plurality of samples. The ECG delineation model may be a deep learning model learned using ECG data containing one or more labeling pairs among the ECG delineation information as a learning set, but the present disclosure is not limited thereto.

The plurality of divided ECG data samples 412 may be analyzed by the ECG delineation model 420 to generate ECG delineation information 431 corresponding to each of the plurality of samples. Each ECG delineation information 431 may include P-wave location information, QRS-complex location information, or T-wave location information.

Meanwhile, the processor 110 may secondarily derive P-wave amplitude information, QRS-complex amplitude information, or T-wave amplitude information from the ECG delineation information 431 for each of the plurality of samples, but the present disclosure is not limited thereto.

Meanwhile, the set 430 of ECG delineation information for the plurality of samples is subjected to an integration process after a smoothing operation by the processor 110. Through integration, single ECG delineation information 440 may be generated. The specific process of smoothing will be described later with reference to FIG. 5.

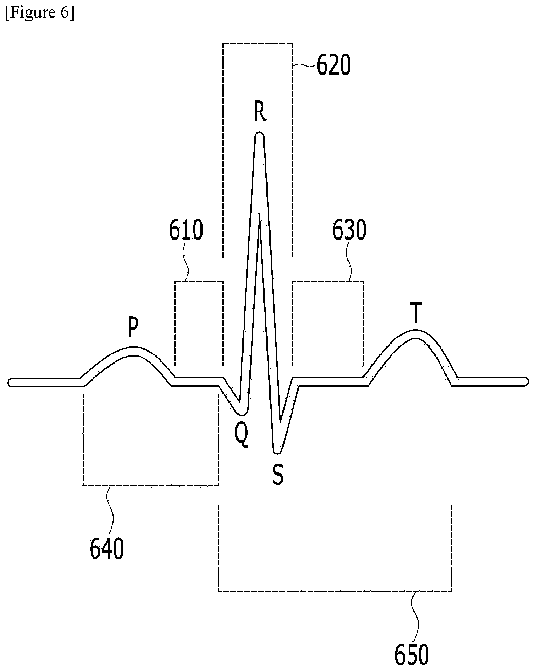

Meanwhile, ECG characteristics may be extracted from the ECG delineation information 440. A detailed description of the electrocardiogram characteristics will be made later with reference to FIG. 6. The integrated ECG delineation information 440 may include multiple heartbeats, and values of the ECG characteristics may vary slightly for each heartbeat. Therefore, in order to smoothly diagnose the patient, the ECG characteristics may be extracted in units of the heartbeat from the integrated ECG delineation information, and representative values of the ECG characteristics may be extracted therefrom. For example, in order to extract the representative values, an average of each heartbeat-unit ECG characteristic is calculated, then characteristics that are further away than a multiple of the standard deviation of the heartbeat-unit ECG characteristic are excluded from the average, and then the average is calculated again to use the average value as the representative value of each ECG characteristic.

Meanwhile, in the above example, when there is an abnormality in heartbeat rhythm or heart disease, the method for extracting the ECG characteristics may vary. For example, when the ECG is measured from a patient with atrial fibrillation, a peak point (R-peak) of the R wave among the ECG characteristics does not exist in the patient's ECG, so a value of a PR interval should not be defined among the ECG characteristics. Therefore, in this case, when a ratio of the number of P-waves and the number of peak points of the R wave is calculated and does not exceed a threshold, it may be defined that there is no PR-interval among the ECG characteristics.

FIG. 5 is a conceptual diagram illustrating one of smoothing processes of the ECG delineation information according to an embodiment of the present disclosure. Referring to FIG. 5, the processor 110 may smooth the ECG delineation information for a plurality of samples in order to correct output noise of the measurement equipment for the ECG delineation information corresponding to each lead. For example, the computing device 100 may designate a window having a fixed sample length with respect to ECG delineation information generated for each lead and smooth the ECG delineation information by a method of applying the mode to all windows among result values to the window, but the present disclosure is not limited thereto.

FIG. 6 is a conceptual diagram showing ECG characteristics that may be extracted from general ECG delineation data. The ECG characteristics, as information on a segment area which becomes an indicator for determining whether the heartbeat rhythm is abnormal or whether there is a heart disease, may include RR-interval, QRS duration 620, QT interval, 650, or QT-corrected interval, but the present disclosure is not limited thereto.

In the meantime, according to an embodiment of the present disclosure, a computer readable medium storing a data structure is disclosed.

The data structure may refer to organization, management, and storage of data that enable efficient access and modification of data. The data structure may refer to organization of data for solving a specific problem (for example, data search, data storage, and data modification in the shortest time). The data structure may also be defined with a physical or logical relationship between the data elements designed to support a specific data processing function. A logical relationship between data elements may include a connection relationship between user defined data elements. A physical relationship between data elements may include an actual relationship between the data elements physically stored in a computer readable storage medium (for example, a permanent storage device). In particular, the data structure may include a set of data, a relationship between data, and a function or a command applicable to data. Through the effectively designed data structure, the computing device may perform a calculation while minimally using resources of the computing device. In particular, the computing device may improve efficiency of calculation, reading, insertion, deletion, comparison, exchange, and search through the effectively designed data structure.

The data structure may be divided into a linear data structure and a non-linear data structure according to the form of the data structure. The linear data structure may be the structure in which only one data is connected after one data. The linear data structure may include a list, a stack, a queue, and a deque. The list may mean a series of dataset in which order exists internally. The list may include a linked list. The linked list may have a data structure in which data is connected in a method in which each data has a pointer and is linked in a single line. In the linked list, the pointer may include information about the connection with the next or previous data. The linked list may be expressed as a single linked list, a double linked list, and a circular linked list according to the form. The stack may have a data listing structure with limited access to data. The stack may have a linear data structure that may process (for example, insert or delete) data only at one end of the data structure. The data stored in the stack may have a data structure (Last In First Out, LIFO) in which the later the data enters, the sooner the data comes out. The queue is a data listing structure with limited access to data, and may have a data structure (First In First Out, FIFO) in which the later the data is stored, the later the data comes out, unlike the stack. The deque may have a data structure that may process data at both ends of the data structure.

The non-linear data structure may be the structure in which the plurality of data is connected after one data. The non-linear data structure may include a graph data structure. The graph data structure may be defined with a vertex and an edge, and the edge may include a line connecting two different vertexes. The graph data structure may include a tree data structure. The tree data structure may be the data structure in which a path connecting two different vertexes among the plurality of vertexes included in the tree is one. That is, the tree data structure may be the data structure in which a loop is not formed in the graph data structure.

Throughout the present specification, a calculation model, a nerve network, the network function, and the neural network may be used with the same meaning. Hereinafter, the terms of the calculation model, the nerve network, the network function, and the neural network are unified and described with a neural network. The data structure may include a neural network. Further, the data structure including the neural network may be stored in a computer readable medium. The data structure including the neural network may also include preprocessed data for processing by the neural network, data input to the neural network, a weight of the neural network, a hyper-parameter of the neural network, data obtained from the neural network, an active function associated with each node or layer of the neural network, and a loss function for training of the neural network. The data structure including the neural network may include predetermined configuration elements among the disclosed configurations. That is, the data structure including the neural network may include the entirety or a predetermined combination of pre-processed data for processing by neural network, data input to the neural network, a weight of the neural network, a hyper parameter of the neural network, data obtained from the neural network, an active function associated with each node or layer of the neural network, and a loss function for training the neural network. In addition to the foregoing configurations, the data structure including the neural network may include predetermined other information determining a characteristic of the neural network. Further, the data structure may include all type of data used or generated in a computation process of the neural network, and is not limited to the foregoing matter. The computer readable medium may include a computer readable recording medium and/or a computer readable transmission medium. The neural network may be formed of a set of interconnected calculation units which are generally referred to as "nodes". The "nodes" may also be called "neurons." The neural network consists of one or more nodes.

The data structure may include data input to the neural network. The data structure including the data input to the neural network may be stored in the computer readable medium. The data input to the neural network may include training data input in the training process of the neural network and/or input data input to the training completed neural network. The data input to the neural network may include data that has undergone pre-processing and/or data to be pre-processed. The pre-processing may include a data processing process for inputting data to the neural network. Accordingly, the data structure may include data to be pre-processed and data generated by the pre-processing. The foregoing data structure is merely an example, and the present disclosure is not limited thereto.

The data structure may include a weight of the neural network (in the present specification, weights and parameters may be used with the same meaning), Further, the data structure including the weight of the neural network may be stored in the computer readable medium. The neural network may include a plurality of weights. The weight is variable, and in order for the neural network to perform a desired function, the weight may be varied by a user or an algorithm. For example, when one or more input nodes are connected to one output node by links, respectively, the output node may determine a data value output from the output node based on values input to the input nodes connected to the output node and the weight set in the link corresponding to each of the input nodes. The foregoing data structure is merely an example, and the present disclosure is not limited thereto.

For a non-limited example, the weight may include a weight varied in the neural network training process and/or the weight when the training of the neural network is completed. The weight varied in the neural network training process may include a weight at a time at which a training cycle starts and/or a weight varied during a training cycle. The weight when the training of the neural network is completed may include a weight of the neural network completing the training cycle. Accordingly, the data structure including the weight of the neural network may include the data structure including the weight varied in the neural network training process and/or the weight when the training of the neural network is completed. Accordingly, it is assumed that the weight and/or a combination of the respective weights are included in the data structure including the weight of the neural network. The foregoing data structure is merely an example, and the present disclosure is not limited thereto.

The data structure including the weight of the neural network may be stored in the computer readable storage medium (for example, a memory and a hard disk) after undergoing a serialization process. The serialization may be the process of storing the data structure in the same or different computing devices and converting the data structure into a form that may be reconstructed and used later. The computing device may serialize the data structure and transceive the data through a network. The serialized data structure including the weight of the neural network may be reconstructed in the same or different computing devices through deserialization. The data structure including the weight of the neural network is not limited to the serialization. Further, the data structure including the weight of the neural network may include a data structure (for example, in the non-linear data structure, B-Tree, Trie, m-way search tree, AVL tree, and Red-Black Tree) for improving efficiency of the calculation while minimally using the resources of the computing device. The foregoing matter is merely an example, and the present disclosure is not limited thereto.

The data structure may include a hyper-parameter of the neural network. The data structure including the hyper-parameter of the neural network may be stored in the computer readable medium. The hyper-parameter may be a variable varied by a user. The hyper-parameter may include, for example, a learning rate, a cost function, the number of times of repetition of the training cycle, weight initialization (for example, setting of a range of a weight value to be weight-initialized), and the number of hidden units (for example, the number of hidden layers and the number of nodes of the hidden layer). The foregoing data structure is merely an example, and the present disclosure is not limited thereto.

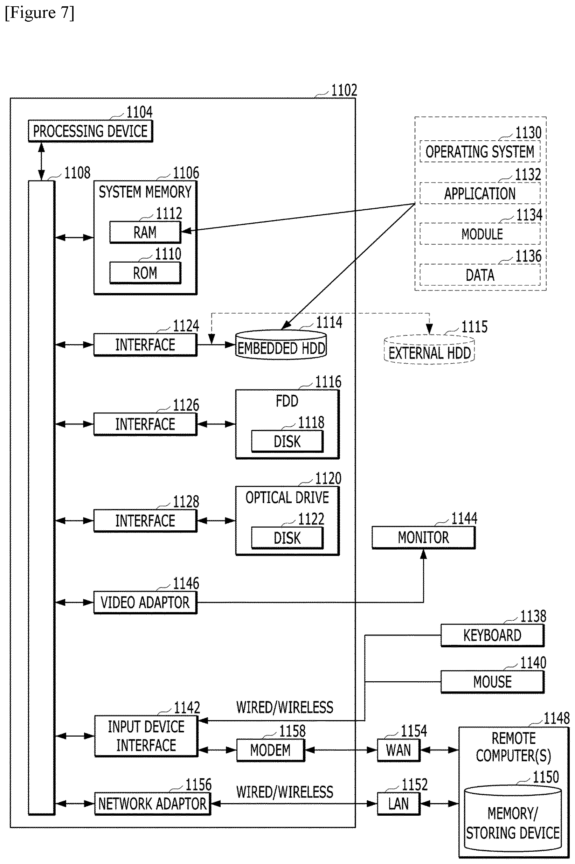

FIG. 7 is a simple and general schematic diagram illustrating an example of a computing environment in which the embodiments of the present disclosure are implementable.

The present disclosure has been described as being generally implementable by the computing device, but those skilled in the art will appreciate well that the present disclosure is combined with computer executable commands and/or other program modules executable in one or more computers and/or be implemented by a combination of hardware and software.

In general, a program module includes a routine, a program, a component, a data structure, and the like performing a specific task or implementing a specific abstract data form. Further, those skilled in the art will well appreciate that the method of the present disclosure may be carried out by a personal computer, a hand-held computing device, a microprocessor-based or programmable home appliance (each of which may be connected with one or more relevant devices and be operated), and other computer system configurations, as well as a single-processor or multiprocessor computer system, a mini computer, and a main frame computer.

The embodiments of the present disclosure may be carried out in a distribution computing environment, in which certain tasks are performed by remote processing devices connected through a communication network. In the distribution computing environment, a program module may be located in both a local memory storage device and a remote memory storage device.

The computer generally includes various computer readable media. The computer accessible medium may be any type of computer readable medium, and the computer readable medium includes volatile and non-volatile media, transitory and non-transitory media, and portable and non-portable media. As a non-limited example, the computer readable medium may include a computer readable storage medium and a computer readable transport medium. The computer readable storage medium includes volatile and non-volatile media, transitory and non-transitory media, and portable and non-portable media constructed by a predetermined method or technology, which stores information, such as a computer readable command, a data structure, a program module, or other data. The computer readable storage medium includes a RAM, a Read Only Memory (ROM), an Electrically Erasable and Programmable ROM (EEPROM), a flash memory, or other memory technologies, a Compact Disc (CD)-ROM, a Digital Video Disk (DVD), or other optical disk storage devices, a magnetic cassette, a magnetic tape, a magnetic disk storage device, or other magnetic storage device, or other predetermined media, which are accessible by a computer and are used for storing desired information, but is not limited thereto.

The computer readable transport medium generally implements a computer readable command, a data structure, a program module, or other data in a modulated data signal, such as a carrier wave or other transport mechanisms, and includes all of the information transport media. The modulated data signal means a signal, of which one or more of the characteristics are set or changed so as to encode information within the signal. As a non-limited example, the computer readable transport medium includes a wired medium, such as a wired network or a direct-wired connection, and a wireless medium, such as sound, Radio Frequency (RF), infrared rays, and other wireless media. A combination of the predetermined media among the foregoing media is also included in a range of the computer readable transport medium.

An illustrative environment 1100 including a computer 1102 and implementing several aspects of the present disclosure is illustrated, and the computer 1102 includes a processing device 1104, a system memory 1106, and a system bus 1108. The system bus 1108 connects system components including the system memory 1106 (not limited) to the processing device 1104. The processing device 1104 may be a predetermined processor among various commonly used processors. A dual processor and other multiprocessor architectures may also be used as the processing device 1104.

The system bus 1108 may be a predetermined one among several types of bus structure, which may be additionally connectable to a local bus using a predetermined one among a memory bus, a peripheral device bus, and various common bus architectures. The system memory 1106 includes a ROM 1110, and a RAM 1112. A basic input/output system (BIOS) is stored in a non-volatile memory 1110, such as a ROM, an EPROM, and an EEPROM, and the BIOS includes a basic routing helping a transport of information among the constituent elements within the computer 1102 at a time, such as starting. The RAM 1112 may also include a high-rate RAM, such as a static RAM, for caching data.

The computer 1102 also includes an embedded hard disk drive (HDD) 1114 (for example, enhanced integrated drive electronics (EIDE) and serial advanced technology attachment (SATA)) - the embedded HDD 1114 being configured for exterior mounted usage within a proper chassis (not illustrated) - a magnetic floppy disk drive (FDD) 1116 (for example, which is for reading data from a portable diskette 1118 or recording data in the portable diskette 1118), and an optical disk drive 1120 (for example, which is for reading a CD-ROM disk 1122, or reading data from other high-capacity optical media, such as a DVD, or recording data in the high-capacity optical media). A hard disk drive 1114, a magnetic disk drive 1116, and an optical disk drive 1120 may be connected to a system bus 1108 by a hard disk drive interface 1124, a magnetic disk drive interface 1126, and an optical drive interface 1128, respectively. An interface 1124 for implementing an outer mounted drive includes, for example, at least one of or both a universal serial bus (USB) and the Institute of Electrical and Electronics Engineers (IEEE) 1394 interface technology.

The drives and the computer readable media associated with the drives provide non-volatile storage of data, data structures, computer executable commands, and the like. In the case of the computer 1102, the drive and the medium correspond to the storage of random data in an appropriate digital form. In the description of the computer readable media, the HDD, the portable magnetic disk, and the portable optical media, such as a CD, or a DVD, are mentioned, but those skilled in the art will well appreciate that other types of computer readable media, such as a zip drive, a magnetic cassette, a flash memory card, and a cartridge, may also be used in the illustrative operation environment, and the predetermined medium may include computer executable commands for performing the methods of the present disclosure.

A plurality of program modules including an operation system 1130, one or more application programs 1132, other program modules 1134, and program data 1136 may be stored in the drive and the RAM 1112. An entirety or a part of the operation system, the application, the module, and/or data may also be cached in the RAM 1112. It will be well appreciated that the present disclosure may be implemented by several commercially usable operation systems or a combination of operation systems.

A user may input a command and information to the computer 1102 through one or more wired/wireless input devices, for example, a keyboard 1138 and a pointing device, such as a mouse 1140. Other input devices (not illustrated) may be a microphone, an IR remote controller, a joystick, a game pad, a stylus pen, a touch screen, and the like. The foregoing and other input devices are frequently connected to the processing device 1104 through an input device interface 1142 connected to the system bus 1108, but may be connected by other interfaces, such as a parallel port, an IEEE 1394 serial port, a game port, a USB port, an IR interface, and other interfaces.

A monitor 1144 or other types of display devices are also connected to the system bus 1108 through an interface, such as a video adaptor 1146. In addition to the monitor 1144, the computer generally includes other peripheral output devices (not illustrated), such as a speaker and a printer.

The computer 1102 may be operated in a networked environment by using a logical connection to one or more remote computers, such as remote computer(s) 1148, through wired and/or wireless communication. The remote computer(s) 1148 may be a work station, a computing device computer, a router, a personal computer, a portable computer, a microprocessor-based entertainment device, a peer device, and other general network nodes, and generally includes some or an entirety of the constituent elements described for the computer 1102, but only a memory storage device 1150 is illustrated for simplicity. The illustrated logical connection includes a wired/wireless connection to a local area network (LAN) 1152 and/or a larger network, for example, a wide area network (WAN) 1154. The LAN and WAN networking environments are general in an office and a company, and make an enterprise-wide computer network, such as an Intranet, easy, and all of the LAN and WAN networking environments may be connected to a worldwide computer network, for example, the Internet.

When the computer 1102 is used in the LAN networking environment, the computer 1102 is connected to the local network 1152 through a wired and/or wireless communication network interface or an adaptor 1156. The adaptor 1156 may make wired or wireless communication to the LAN 1152 easy, and the LAN 1152 also includes a wireless access point installed therein for the communication with the wireless adaptor 1156. When the computer 1102 is used in the WAN networking environment, the computer 1102 may include a modem 1158, is connected to a communication computing device on a WAN 1154, or includes other means setting communication through the WAN 1154 via the Internet. The modem 1158, which may be an embedded or outer-mounted and wired or wireless device, is connected to the system bus 1108 through a serial port interface 1142. In the networked environment, the program modules described for the computer 1102 or some of the program modules may be stored in a remote memory/storage device 1150. The illustrated network connection is illustrative, and those skilled in the art will appreciate well that other means setting a communication link between the computers may be used.

The computer 1102 performs an operation of communicating with a predetermined wireless device or entity, for example, a printer, a scanner, a desktop and/or portable computer, a portable data assistant (PDA), a communication satellite, predetermined equipment or place related to a wirelessly detectable tag, and a telephone, which is disposed by wireless communication and is operated. The operation includes a wireless fidelity (Wi-Fi) and Bluetooth wireless technology at least. Accordingly, the communication may have a pre-defined structure, such as a network in the related art, or may be simply ad hoc communication between at least two devices.

The Wi-Fi enables a connection to the Internet and the like even without a wire. The Wi-Fi is a wireless technology, such as a cellular phone, which enables the device, for example, the computer, to transmit and receive data indoors and outdoors, that is, in any place within a communication range of a base station. A Wi-Fi network uses a wireless technology, which is called IEEE 802.11 (a, b, g, etc.) for providing a safe, reliable, and high-rate wireless connection. The Wi-Fi may be used for connecting the computer to the computer, the Internet, and the wired network (IEEE 802.3 or Ethernet is used). The Wi-Fi network may be operated at, for example, a data rate of 11 Mbps (802.11a) or 54 Mbps (802.11b) in an unauthorized 2.4 and 5 GHz wireless band, or may be operated in a product including both bands (dual bands).

Those skilled in the art may appreciate that information and signals may be expressed by using predetermined various different technologies and techniques. For example, data, indications, commands, information, signals, bits, symbols, and chips referable in the foregoing description may be expressed with voltages, currents, electromagnetic waves, magnetic fields or particles, optical fields or particles, or a predetermined combination thereof.

Those skilled in the art will appreciate that the various illustrative logical blocks, modules, processors, means, circuits, and algorithm operations described in relationship to the embodiments disclosed herein may be implemented by electronic hardware (for convenience, called "software" herein), various forms of program or design code, or a combination thereof. In order to clearly describe compatibility of the hardware and the software, various illustrative components, blocks, modules, circuits, and operations are generally illustrated above in relation to the functions of the hardware and the software. Whether the function is implemented as hardware or software depends on design limits given to a specific application or an entire system. Those skilled in the art may perform the function described by various schemes for each specific application, but it shall not be construed that the determinations of the performance depart from the scope of the present disclosure.

Various embodiments presented herein may be implemented by a method, a device, or a manufactured article using a standard programming and/or engineering technology. A term "manufactured article" includes a computer program, a carrier, or a medium accessible from a predetermined computer-readable storage device. For example, the computer-readable storage medium includes a magnetic storage device (for example, a hard disk, a floppy disk, and a magnetic strip), an optical disk (for example, a CD and a DVD), a smart card, and a flash memory device (for example, an EEPROM, a card, a stick, and a key drive), but is not limited thereto. Further, various storage media presented herein include one or more devices and/or other machine-readable media for storing information.

It shall be understood that a specific order or a hierarchical structure of the operations included in the presented processes is an example of illustrative accesses. It shall be understood that a specific order or a hierarchical structure of the operations included in the processes may be rearranged within the scope of the present disclosure based on design priorities. The accompanying method claims provide various operations of elements in a sample order, but it does not mean that the claims are limited to the presented specific order or hierarchical structure.

The description of the presented embodiments is provided so as for those skilled in the art to use or carry out the present disclosure. Various modifications of the embodiments may be apparent to those skilled in the art, and general principles defined herein may be applied to other embodiments without departing from the scope of the present disclosure. Accordingly, the present disclosure is not limited to the embodiments suggested herein, and shall be interpreted within the broadest meaning range consistent to the principles and new characteristics presented herein.

## Claims

1. A method performed by one or more processors of a computing device, the method comprising:
receiving one or more ECG data;
generating a plurality of ECG delineation information corresponding to a plurality of samples having a predetermined time length, from the one or more received ECG data through an ECG delineation model; and
integrating the plurality of ECG delineation information.

2. The method of claim 1, wherein the receiving of the ECG data includes:
receiving the one or more ECG data from one of a plurality of different types of ECG measurement equipment.

3. The method of claim 1, wherein the predetermined time length is determined independently of measurement time length or a number of leads of an ECG measurement equipment.

4. The method of claim 1, wherein the generating of the plurality of ECG delineation information corresponding to the plurality of samples having the predetermined time length, from the one or more received ECG data through the ECG delineation model includes:
confirming a measurement time length and a number of leads of an ECG measurement equipment,
generating the plurality of samples by dividing the one or more ECG data based on the predetermined time length, and
generating respective ECG delineation information corresponding to the plurality of samples through the ECG delineation model.

5. The method of claim 1, wherein the ECG delineation information includes at least one of:
location information of a P-wave,
location information of a QRS-complex, or
location information of a T-wave, and
wherein the processor derives, from the ECG delineation information, at least one of:
amplitude information of the P-wave,
amplitude information of the QRS-complex, or
amplitude information of the T-wave.

6. The method of claim 1, wherein the integrating of the plurality of ECG delineation information includes:
generating smoothed ECG delineation information for the plurality of samples, and
integrating the smoothed ECG delineation information for the plurality of samples.

7. The method of claim 6, wherein the generating of the smoothed ECG delineation information for the plurality of samples further includes:
deriving a representative value among result values of the ECG delineation information for the plurality of samples within a fixed time interval.

8. The method of claim 6, wherein the integrating of the smoothed ECG delineation information for the plurality of samples further includes:
deriving a representative value among the result values of the smoothed ECG delineation information.

9. The method of claim 1, further comprising:
extracting ECG characteristics from the integrated ECG delineation information,
wherein the extracting of ECG characteristics from the integrated ECG delineation information includes:
dividing the integrated ECG delineation information based on one heart beat time,
extracting each ECG characteristic from the ECG delineation information divided based on the one heart beat time, and
deriving a representative value of each extracted ECG characteristic.

10. The method of claim 9, wherein the ECG characteristics include at least one of:
an RR-interval,
a QRS duration,
a QT interval, or
a QT-corrected interval.

11. The method of claim 1, further comprising:
generating a user interface (UI) including information on the ECG delineation information,
wherein the user interface includes:
a first area displaying ECG data measured by an ECG measurement equipment,
a second area displaying the ECG delineation information, and
a third area displaying a value of an ECG characteristic.

12. A computer program stored in a computer readable storage medium, the computer program including instructions which cause a computing device to perform operations, wherein the operations comprise:
an operation of receiving one or more ECG data;
an operation of generating ECG delineation information corresponding to a plurality of samples having predetermined time lengths, from the one or more received ECG data through an ECG delineation model; and
an operation of integrating the plurality of ECG delineation information.

13. A computing device comprising:
a processor including one or more cores;
a network unit receiving one or more ECG data; and
a memory,
wherein the processor is configured to:
receive one or more ECG data,
generate ECG delineation information corresponding to a plurality of samples having predetermined time lengths, from the one or more received ECG data through an ECG delineation model, and
integrate the plurality of ECG delineation information.
